# EUROPEAN PATENT APPLICATION

(11) **EP 3 501 377 A1**
(43) Date of publication of application: **26.06.2019**
(21) Application number: 17209454.2
(22) Date of filing: 21.12.2017
(51) Int. Cl.: A61B 5/00, A61B 5/107, A61B 5/11

(54) **WEARABLE DEVICE AND METHOD FOR MONITORING A JOINT OF A HUMAN OR ANIMAL BODY**

(71) Applicant: Hollander, Dirk A., 61476 Kronberg (DE); Boles, Ernest, 61352 Bad Homburg (DE)
(72) Inventor: Hollander, Dirk A., 61476 Kronberg (DE); Boles, Ernest, 61352 Bad Homburg (DE)
(74) Representative: Jacobi, Markus Alexander

(57) **Abstract**

A wearable device for monitoring a joint of a human or animal body is provided comprising a bandage for protecting and/or supporting a joint of the body, the joint being located between a first and a second body part. The bandage comprises a first bandage section which is attachable to the first body part, a second bandage section which is attachable to the second body part and a third bandage section which at least partially covers the joint. The wearable device further comprises at least one sensor for acquiring sensor data which is characteristic for the condition of the joint, and a communication device for wireless transmission of the acquired sensor data. In addition, the wearable device comprises at least one sensor which is configured for measuring a swelling of the joint and at least one sensor which is configured for measuring an orientation and/or a motion of the first body part relative to the second body part.

Further aspects of the invention relate to a system comprising such a wearable device and to a method for monitoring the condition of a joint of a human or animal body.

## Description

The invention relates to a wearable device for monitoring a joint of a human or animal body which comprises at least one sensor for acquiring sensor data which is characteristic for the condition of the joint. Further aspects of the invention relate to a system comprising such a wearable device and to a method for monitoring the condition of a joint of a human or animal body.

Wearable devices which are configured for monitoring health related parameters of a patient are known in the art. Document US 2012/084053 discloses a portable monitoring device which is adapted to monitor the physical activity of a user and other activity-related metrics. The monitoring device includes a housing which allows the device to be coupled to the user, for example to the arm, wrist, ankle, waist or foot of the user. The monitoring device may comprise sensors such as accelerometers, gyroscope, compass, altimeter and an optical heart rate sensor. Further, the monitoring device may be adapted to calculate other parameters such as calorie burn.

Document DE 10 2015 100 795 discloses a device for determining data on movements of a joint which connects two body parts. The device comprises two motion sensors which may be attached to the two respective body parts. The absolute and/or relative movement of the two body parts is derived from data acquired by means of the motion sensors.

In advance of and/or after a joint operation it is advantageous to monitor the patient in order to ensure that the treating physician has reliable information and data to ensure that the patient and the respective joint receive the optimal treatment. Further, monitoring of a joint is also advantageous in physiotherapeutic treatment of a joint.

Many different parameters should be monitored in order to reliably assess the condition of the respective joint. Most of the known wearable devices monitor only few activity related parameters such as heart rate and activity or are specifically configured to measure a specific parameter such as the motion of a joint. There is a need for a wearable device which provides all parameters which are required for assessing the condition of a joint.

The problem is solved by providing a wearable device for monitoring a joint of a human or animal body comprising a bandage for protecting and/or supporting a joint of the body, the joint being located between a first and a second body part. The bandage comprises a first bandage section which is attachable to the first body part, a second bandage section which is attachable to the second body part and a third bandage section which at least partially covers the joint. The wearable device further comprises at least one sensor for acquiring sensor data which is characteristic for the condition of the joint, and a communication device for wireless transmission of the acquired sensor data. In addition, the wearable device comprises at least one sensor which is configured for measuring a swelling of the joint and at least one sensor which is configured for measuring an orientation and/or a motion of the first body part relative to the second body part.

The wearable device may include further components in addition to the sensors and the communication device, in particular electronic components such as a battery, on/off switch, charging port and/or processing means for controlling the measurements and/or analyzing the acquired data.

The bandage, in particular the first section and/or the second section of the bandage, may be fixed to the respective body part. For securing the bandage, a fastener mechanism such as Velcro (hook and loop fasteners), snap fasteners, zippers or laces may be provided. Further, combinations of two or more of these fastener mechanisms may be used.

Preferably, the used fastener mechanism allows for size adjustment of the bandage and/or the respective section of the bandage in order to account for different swelling of the respective body parts. For example, Velcro fasteners allow for an easy size adjustment.

If snap fasteners are used, multiple snap fasteners may be provided to allow fastening of the bandage and/or of the respective bandage section in more than one position to account for different swelling of a body part.

Preferably, the fastener mechanism allows the bandage to be opened completely so that the bandage may be directly removed without having to slide the bandage over the respective body part. Such a fastener mechanism makes it easy to put the bandage on and to take the bandage off.

In interventions involving a joint, the use of postsurgical drainages such as drainage tubes is standard practice in order to minimize complications such as postoperative hematoma and/or infections of the wound. In order to facilitate the use of the bandage directly after an intervention, the fastener mechanism of the bandage may include an opening in order to pass through a drainage tube.

The bandage may also be constructed like a sock or a tube and the bandage may be pushed or slid over the respective joint. An elastic band and/or Velcro may be used for securing the first, second and/or third section of the bandage to the respective body parts.

The material of the bandage preferably includes a mixture of elastic and non-elastic materials. Further, the material of the bandage offers breathability, is easy to clean and is moisture-permeable. Preferably, the bandage is made of a textile material which includes elastic and non-elastic fibers. Washable textile materials are preferred. Suitable materials for the bandage include textile materials comprising a thermoplastic composition, based e.g. on polyamide and/or polyester, such as a blend of polyamide fibers, polyester fibers and/or spandex (polyether-polyurethane) fibers.

The bandage may be constructed as a single piece or may consist of more than one piece.

Preferably, the bandage comprises a wound dressing which is detachably connected to the third section of the bandage and configured to treat and/or protect a wound at the joint. The wound dressing is detachably connected to the bandage, for example by means of Velcro or snap fasteners.

The shape of the wound dressing is chosen such that after the intervention a wound is covered and protected. The detachably connected wound dressing allows the wound dressing to be cleaned and/or exchanged independently from the bandage. The part of the bandage which is in the section of the wound can be equipped with a pharmaceutical product, such as an antiseptic and/or antibiotic component.

The bandage may comprise at least one pocket. The pocket may be configured for storage of a pharmaceutical component (medicine) or for inserting other elements.

Preferably, the bandage or parts of the bandage which are in contact with the human or the animal body are detachably connected to the wearable device so that they may be removed for cleaning or in order to be exchanged. This allows the bandage to be thoroughly cleaned without harming the sensors or further electronic components.

Preferably, the wearable device is waterproof so that the entire wearable device may be washed and/or cleaned. In such an embodiment, the sensors and further electronic components are constructed and arranged such that washing of the wearable device is possible without having to remove the electronic parts.

If the wearable device is intended to be worn by a human, the bandage is preferably configured as a bandage for the knee, the ankle, the hip, the wrist, the elbow or the shoulder of a patient. The wearable device may be configured to be worn by an animal such as a horse. In case the wearable device is tended to be worn by a horse, the bandage is preferably configured for the knee, fetlock joint or the hock.

Preferably, the wearable device comprises in the range of from one to five sensors which are configured for measuring a swelling of the joint and which are arranged at different positions in the third bandage section. The at least one sensor configured for measuring a swelling of the joint is for example a strain sensor which is adapted for at least partial wrapping around the joint such that a change of girth of the joint is detected as a change of strain.

Alternatively, the at least one sensor configured for measuring a swelling of the joint may be configured as a flex sensor which detects bending, wherein the sensor is arranged such that it is at least partially wrapped around the joint so that a change of girth of the joint is detected as a change of bending radius of the flex sensor.

The sensor configured for measuring a swelling is preferably arranged in the third bandage section which covers at least a part of the respective joint. Several of said sensors may be arranged in the third bandage section, especially if the joint is large compared to the size of the sensor.

Preferably, the at least one sensor configured for measuring an orientation and/or a motion of the first body part relative to the second body part comprises motion detectors which are located in at least two of the three bandage sections.

The motion sensors are each capable of detecting a change in positon or a change of orientation when the body part to which the respective bandage section is attached is moved.
When the detected changes in positions and/or orientation of sensors arranged in different bandage sections are compared, a relative movement of one bandage section to the respective other bandage section can be determined. In particular, the motion sensors may be configured to determine a range of motion of the respective joint. This range of motion may change depending on the condition of the joint.

For example, if both the first and the second bandage section each comprise motion sensors capable of detecting changes in orientation, a change in relative orientation of the first bandage section to the second bandage section may be determined. This data allows for determining a change of relative orientation of the two body parts which are connected by the joint as each of the first and the second bandage sections are attached to said body parts. Thus, the data acquired by the motion detectors may be used for monitoring the motion of the joint. By monitoring the motion of the joint over time, the range of motion of the respective joint may be determined by analyzing the extreme values of the detected range of motion.

Preferably, the motion detectors are configured as accelerometers and/or gyroscopes.

Accelerometers measure the change in position of an object (acceleration) and gyroscopes measure a change in direction of an object. By means of integration of the data acquired by said sensors, the present position and/or orientation may be calculated with respect to an initial position and orientation.

The initial position and/or orientation may be an arbitrary position/orientation such as the position and/or orientation of the respective motion sensor when the wearable device is powered on. Further, when analyzing the motion, especially extension or flexion, of the joint, the initial position and/or orientation may be set to one of the detected extreme positions of the range of motion of the respective joint. For example, in case of a knee the initial position may be set to the position and/or orientation of the motion sensors with the knee in full extension.

The accelerometers and/or gyroscopes are preferably configured as microelectromechanical systems (MEMS). The accelerometers and/or gyroscopes are preferably provided in the form of an integrated system which comprises several accelerometers and/or gyroscopes in a single system.

The wearable device is preferably configured to acquire further parameters related to the condition of the joint and/or of the patient wearing the device. Preferably, the wearable device comprises at least one sensor configured for measuring the temperature of the joint and/or at least one sensor configured for measuring heart rate and/or at least one sensor configured for measuring blood pressure and/or at least one sensor configured for measuring oxygen saturation and/or at least one sensor configured for measuring activity.

A sensor may be configured for measuring a single one of said parameters or a sensor may be configured to measure two or more of said parameters. For example, a sensor may be configured to acquire both oxygen saturation and heart rate.

Suitable sensors for measuring activity include by way of example motion sensors such as gyroscopes and accelerometers and position sensors, especially receivers for global navigation satellite systems (GNSS) such as GPS receivers.

The motion sensor may detect movement and/or rotation and these parameters may be used to derive a level of activity. For example, walking causes a repetitive up and down motion which allows counting of steps by use of an accelerometer. The GNSS receiver may track the position of the patient and may be used to determine a travelled distance.

Suitable sensors for measuring heart rate may include by way of example optical sensors, electrical sensors and acoustic sensors. An optical sensor may, for example, measure the light reflected by blood vessels beneath the skin. The rate of absorption changes periodically with the heartbeat and may be used to acquire the heart rate. An acoustic sensor may listen to the noise caused by the beating heart and/or the flowing blood and may use a periodic signal to acquire heart rate. An electric sensor may likewise detect an electric signal caused by the beating heart in order to derive the heart rate.

Suitable sensors for measuring oxygen saturation include by way of example optical sensors which measure a change in the spectrum of light reflected by blood vessels beneath the skin.

A suitable sensor for determining blood pressure may include for example an inflatable cuff and a microphone.

Suitable sensors for measuring body temperature include for example thermocouples, thermistors and resistance temperature detectors (RTD).

The respective sensors are preferably connected to the communication device. The communication device may then acquire the respective sensor data and may transmit the data wirelessly.

Preferably, the wearable device further comprises an input device configured to allow a patient to input a value representing a perceived pain, wherein the input device is connected to the communication device and the communication device is configured for wireless transmission of the value. The input device may, for example, be configured as a mechanical slider, a mechanical dial, a touch input device or a keypad comprising one or more buttons. The input device can be considered as a sensor for acquiring the perceived pain of the patient.

Preferably the input device is arranged on one of the three bandage sections so that the input device may be accessed easily. Alternatively, the patient may be provided with an app which may be run on a smart device such as a tablet or a smartphone or a website may be provided which can be accessed by means of an internet enabled device, wherein the app and/or the website is configured to allow the patient to input a value representing the currently perceived pain. Further, the app and/or website may allow the patient to provide further comments which may be made available to his physician.

The input device allows the patient to input the perceived pain which may then be acquired and stored in conjunction with other parameters characterizing the condition of the joint. This allows a physician to receive further input which may be used to adjust a treatment plan. For example, a training plan may be adapted in order to avoid situations which cause the patient pain and/or the prescribed medication such as the dosage of pain killers may be adapted.

The wearable device preferably comprises means for reminding the patient to perform a certain action such as to begin an activity, input the currently perceived pain and/or to take prescribed medicine.

These means may be selected from acoustic, optic and haptic signaling means. Acoustic signaling means may include loudspeakers which may be used to play a sound or a message reminding the patient to perform a certain action. Optic signaling means may include screens, and/or lights such as LEDs which may flash or display a message to remind the patient to perform a certain action. Haptic signaling means may include a vibration motor configured to provide a haptic signal which reminds the patient to perform a certain action.

For configuring a reminder, the wearable device preferably communicates with a smart device such as a tablet or a smartphone and/or with a remote device accessible over the internet. An app which is executed on the smart device or a website provided by the remote device accessible over the internet may then be used to configure a reminder.

The wearable device comprises a communication device for wireless transmission of data acquired by the sensors.

Preferably, the communication device is adapted to communicate via technologies such as wlan, Bluetooth, zigbee, ANT+ and/or cellular networks and is configured to communicate with an application executed on a smart device and/or a remote device accessible over the internet.

Examples for smart devices are mobile devices such as smartphones and tablets. These smart devices include communication hardware which is able to receive the data which is transmitted by the communication device of the wearable device. An app which is configured to be executed on the smart device may be provided which is capable of storing and analyzing the received data. Further, the app may be configured to upload the received data to a remote device accessible over the internet, such as a website or a cloud computing service. Further, the app may be configured for sharing of the received data, for example by means of email.

Examples for a remote device which is accessible over the internet include websites and cloud computing services. The wearable device may be configured to upload acquired sensor data and/or further data such as the value representing the perceived pain to the remote device. The remote device may include an interface for a physician to access the data. For example, a website may be provided which allows the physician to access the acquired data.

The wearable device may further comprise processing means such as a microcontroller or a computer, for example in form of a system-on-chip. The processing means are preferably configured for controlling the sensors and the transmission of data by the communication device. Further, the processing means may be configured to process the acquired data. Alternatively or in addition to processing means of the wearable device, the remote device which is accessible over the internet may be configured for processing of the data received by the wearable device.

When the data is processed or analyzed, the data representing the monitored parameters may be compared with reference values which may have been acquired prior to an intervention. Threshold levels may be defined and certain actions may be triggered when an observed parameter is below or above a predetermined threshold.

Such actions may be selected, for example, from sending a notification to a physician, sharing acquired data with a physician, reminding the patient to perform a certain action and giving recommendations to adjust a treatment plan and combinations of more than one of these actions.

Further, processed and/or unprocessed data acquired by the wearable device may be shared with a healthcare provider such a physician, a physiotherapist and/or a sports team trainer. Sharing of the data may be performed automatically and/or when triggered by the user.

For example, if a certain value for the range of motion is not exceeded after a certain time has passed since the intervention, the physician may be informed. The physician may then review the acquired data and may propose changes to a treatment plan depending on parameters such as activity of the patient, perceived pain and swelling.

A further example is setting of threshold values such that the physician is informed when a temperature of the joint is above 37.5°C and/or the swelling exceeds a predetermined value.

Further, by processing the data, two or more of the acquired parameters may be combined to derive a score which represents the condition of the monitored joint. Further, by processing of the data recommendations may be derived which may be used to adjust a treatment plan.

Processing of the data may also include preparing graphs which show the change of an observed parameter over time. Such a graph may be presented to the patient, for example by means of an app executed on a smart device, or could be made available to a physician on a website.

A further aspect of the invention is to provide a system comprising one of the described wearable devices, wherein the bandage of the wearable device is configured as a bandage for a joint of a lower extremity of the human body, and wherein the system further comprises a sole insert comprising a sensor configured for measuring pressure distribution and the system being configured for determining a gait pattern using the measured pressure distribution.

The system further comprises processing means being configured to determine a weight loading of the joint of the lower extremity, wherein the gait pattern is used as input value.

The joint of the lower extremity may for example be a knee, an ankle or a hip.

The processing means may be associated with the wearable device or the sole. Alternatively, the processing means may be provided in the form of a smart device or a remote device which is accessible over the internet. In this case, the wearable device and/or the sole insert wirelessly transmit data which are then processed by the smart device or the remote device. Further, the gait pattern and/or the weight loading may be used to derive recommendations for the patient.

The analyzed gait pattern, derived data such as the weight loading of the respective joint and/or recommendations may be made available to the patient, for example by means of an app executed on a smart device or by means of a website provided by the remote device.

As already described with respect to the wearable device, thresholds may be defined for the gait pattern and/or the weight load and an action may be triggered if a predetermined threshold is exceeded.

Preferably, the sole insert and the wearable device are configured such that the sole transmits acquired data to the wearable device by means of a low energy and low range communication device.
The wearable device then serves as a relay and transmits the data of the sole insert to the smart device and/or the remote device. The low range communication device may by way of example be configured as a Bluetooth device, zigbee device or ant+ device.

For determining the pressure distribution, the sole insert may comprise pressure sensors. Suitable pressure sensors include, for example, piezoelectric or MEMS type sensors.

A further aspect of the invention is to provide a method for monitoring the condition of a joint of a human or animal body, wherein one of the described wearable devices or one of the described systems is worn by a patient or an animal prior to an intervention for a first time period, wherein reference data is acquired, the reference data at least including a reference range of motion of the joint and a reference swelling of the joint. Further the method includes wearing of the wearable device or the system after an invention for a second time period and/or a third time period, wherein during the second time period and/or third time period sensor data which is characteristic for the condition of the joint is acquired and the condition of the joint is evaluated by comparing the acquired data with the previously acquired reference data, wherein the acquired data at least includes a range of motion of the joint derived from data acquired by means of motion sensors and a swelling of the joint.

It is preferred that at least during the second time period and/or the third time period further data is acquired and is considered for evaluating the condition of the joint, wherein the further data is selected from heart rate, blood pressure, oxygen saturation, temperature of the joint, perceived pain, activity and combinations of at least two of these parameters.

Preferably, the method comprises transmitting of acquired data and/or the evaluated condition of the joint to a smart device of the patient and/or to a remote device.

The wearable device can be used in a wide variety of surgical and non-surgical applications, ranging from the monitoring and/or treatment of the effected joint, as part of a sports regime, physiotherapy or training program to joint surgery or replacement. By way of example, in circumstances requiring surgical intervention, a typical treatment involving a joint has three stages. In a first stage, the pre-operative stage, the wearable device is worn for a first period of time in advance of the surgical intervention. The length of the first time period is typically up to 4 weeks, often up to 6 weeks.

During the first stage, the patient is prepared for the intervention. Preparation involves acquiring reference data of the joint which is stored for later use. Further, the patient performs exercises which are intended to strengthen the joint and in particular the muscles surrounding and supporting the joint.

While the patient wears the wearable device, the joint may be monitored by a physician and/or physiotherapist. Pre-operative data may be recorded which will serve as reference. Further, the wearable device may be used to assess the condition of the respective joint, such as range of motion prior to the intervention. The wearable device may also be used to monitor the activity of the patient prior to the intervention. In particular, the physician may monitor the compliance of the patient with prescribed exercises for improving the strength of the muscles supporting the joint. The acquired data may be used to assist the patient prior to the intervention by providing feedback to the patient whether the training is sufficient or not. Further, the exercise regimen may be adjusted depending on the condition of the joint. For example, a training plan may be adapted in order to avoid situations which cause the patient pain and/or the prescribed medication such as the dosage of pain killers may be adapted.

When preparations have been completed, the intervention takes place at a medical facility.

In a second treatment stage, the post-operative stage, the patient is under supervision in a medical facility or ambulant and the wearable device may be used to monitor respective parameters of the patient and the joint. The wearable device may be worn directly after the intervention. Advantageously, the wearable device allows for continuous monitoring of parameters such as temperature and swelling so that complications may be detected early and necessary countermeasures can be initiated immediately.

The third treatment stage is the rehab stage, which typically begins after drainage has been removed and continues on after the patient is released from the medical facility. Rehab usually includes an exercise regimen to be performed by the patient in order to strengthen muscles supporting the joint and in order to improve the range of motion of the respective joint. The wearable device is used to continue monitoring of the patient. In particular, the effect of rehabilitation on the joint is monitored and feedback may be provided to the patient.

Feedback may be provided, for example, by means of an app which is executed on a smart device of the patient, such as a smartphone or a tablet, or may be provided in form of a website. Feedback may include an indication whether the patient has correctly performed his exercises.

Furthermore, the feedback may include a diagram showing the progress he has made, for example by showing how the range of motion or the overall condition of the joint has improved over time. The overall condition of the joint may be represented by a score which is based on several acquired parameters, for example swelling, temperature and range of motion. By provided such feedback, compliance of the patient with the treatment plan is improved.

The wearable device may be exchanged after each of the stages has ended or the same wearable device may be used for all three stages of treatment. The bandage of the wearable device and/or parts of the wearable device which are in direct contact with the patient may be exchanged or cleaned from time to time.

In each of the stages of treatment, the wearable device allows for early recognition of problems which could otherwise negatively impact the treatment duration.

For example, the physician may use the acquired data to judge the overall condition of the joint over time and may recognize whether the condition is adequate or not. If the condition is inadequate, the wearable device provides the physician with the data required to determine the cause. For example, a low activity shows that the patient does not perform his exercise regimen as prescribed. A temperature above 37.5 °C and/or a swelling may indicate an infection which has to be treated. A high perceived pain level may indicate that the pain medication has to be adjusted.

Further, the physician can compare the present situation with reference data recorded prior to the intervention. Deviations from the reference data may indicate improvement or a problem which has to be addressed.

Countermeasures include adjusting the exercise regimen, adjusting the load during exercise, cooling of the joint, lymph drainage, prescription of antibiotics, dosage adjustment of pain killers and revision surgery. The necessary countermeasures are preferably selected based on the present and previously recorded sensor data.

After the treatment has been completed, the success of the intervention may be assessed. Criteria for a successful treatment may include that parameters such as heart rate and swelling correspond to the pre-operative reference values.

In case of a knee as monitored joint, the range of motion should allow a full extension and at least 100° to 120° bending of the knee. The perceived pain should be less than prior to the intervention.

The wearable device may also be used in non-surgical applications. For example, the wearable device can be used to support diagnostic and therapy.

### Brief description of the Figures:

Figure 1 shows a schematic view of a wearable device for monitoring a knee and
Figure 2 shows a schematic view of a wearable device for monitoring an ankle.

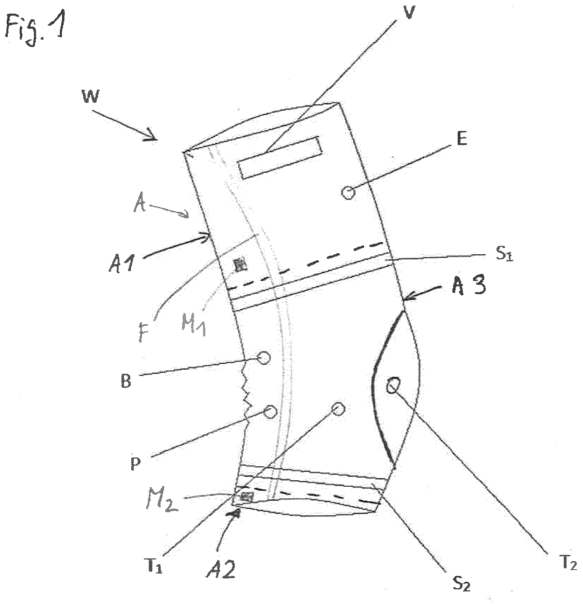

Figure 1 depicts a wearable device W for monitoring a knee. The wearable device W comprises a bandage A having three bandage sections. A first bandage section A1 allows the bandage A to be attached to a first body part. A second bandage section A2 allows the bandage A to be attached to a second body part. In the depicted example embodiment, the joint to be monitored is a knee so that the first body part is an upper leg of a patient and the second body part is a lower leg of a patient. The knee connects the two body parts. A third bandage section A3 is configured to at least partially cover the knee if the wearable device W is worn by a patient.

The bandage A further comprises a fastener mechanism F which allows the bandage A to be opened. The fastener mechanism F is for example a hook and loop fastener such as a Velcro strap.

The wearable device W comprises several sensors S, T, M, B, P which are connected to a communication device E which is adapted to transmit the respective sensor data. The wearable device W comprises a first motion sensor M1 attached to the first bandage section A1 and a second motion sensor M2 attached to the second bandage section A2. By means of the motion sensors M1, M2 a relative orientation of the first bandage section A1 to the second bandage section A2 may be determined. If the respective bandage sections A1 and A2 are attached to the upper leg and the lower leg of a patient, respectively, the range of motion of the knee including extension and flexion may be determined.

Two temperature sensors T1, T2 are arranged in the third bandage section. A first temperature sensor T1 is arranged so that it is located close to the side of the knee when the wearable device W is worn. A second temperature sensor T2 is arranged such that it is located near the patella when the wearable device W is worn.

For detection of a swelling of the knee, two swelling sensors S1, and S2 are arranged in the third bandage section A3. A first swelling sensor S1 is located above the patella, a second swelling sensor S2 is arranged such that it is located below the patella when the wearable device W is worn.

Further sensors such as a blood pressure sensor B and a heart rate sensor P may also be arranged in the third bandage section A3.

Further, the wearable device W comprises an input device V which is in the depicted example constructed as an analogue slider. A patient may use the analogue slider to input a value which represents the level of perceived pain. The input device V is connected to the communication device E so that the value may also be transmitted.

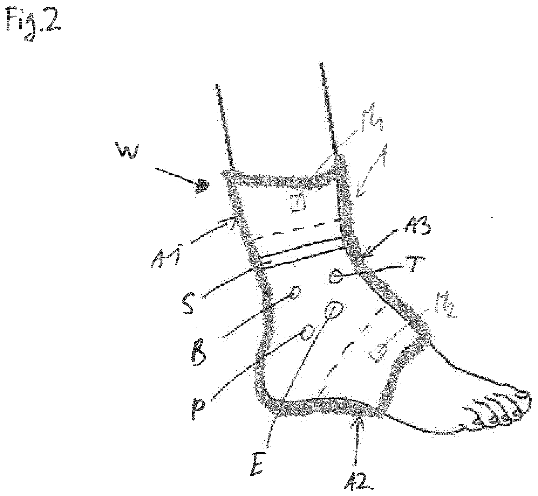

Figure 2 depicts a wearable device W for monitoring an ankle. The wearable device W comprises a bandage A which is configured as a tube which may be sled over the ankle of a patient. As described with respect to figure 1, the bandage A comprises three bandage sections A1, A2 and A3. The first bandage section A1 is adapted to be attached to the lower leg of a patient. The second bandage section A2 is adapted to be attached to a foot of a patient. The third bandage section A3 is adapted to cover at least a part of the ankle of a patient.

The wearable device W comprises several sensors S, T, M, B, P which are connected to a communication device E which is adapted to transmit the respective sensor data. The wearable device W comprises a first motion sensor M1 attached to the first bandage section A1 and a second motion sensor M2 attached to the second bandage section A2. By means of the motion sensors M1, M2 a relative orientation of the first bandage section A1 to the second bandage section A2 may be determined.

If the respective bandage sections A1 and A2 are attached to the lower leg and the foot of a patient, respectively, the range of motion of the ankle may be determined.

In the example embodiment of figure 2, a single temperature sensor T is arranged in the third bandage section. Further, for detection of a swelling of the ankle, a single swelling sensor S is arranged in the third bandage section. Further sensors such as a blood pressure sensor B and a heart rate sensor P may also be arranged in the third bandage section A3.

### List of reference numerals

- W: wearable device
- A: bandage (A1: first bandage section; A2: second bandage section; A3: third bandage section)
- B: blood pressure sensor
- T: temperature sensor (T1: first temperature sensor; T2: second temperature sensor)
- P: heart rate sensor
- S1, S2: swelling sensor
- V: input device with visual analogue scale (VAS)
- E: communication device
- F: fastener mechanism
- M1, M2: motion sensor

## Claims

1. Wearable device for monitoring a joint of a human or animal body comprising a bandage for protecting and/or supporting a joint of the body, the joint being located between a first and a second body part, the bandage comprising a first bandage section which is attachable to the first body part, a second bandage section which is attachable to the second body part and a third bandage section which at least partially covers the joint,
the wearable device further comprising at least one sensor for acquiring sensor data which is characteristic for the condition of the joint, and
a communication device for wireless transmission of the acquired sensor data, **characterized in that**
the wearable device comprises at least one sensor which is configured for measuring a swelling of the joint and at least one sensor which is configured for measuring an orientation and/or a motion of the first body part relative to the second body part.

2. Wearable device according to claim 1, **characterized in that** the wearable device comprises in the range of from one to five sensors which are configured for measuring a swelling of the joint and which are arranged at different positions in the third bandage section.

3. Wearable device according to claims 1 or 2, **characterized in that** the at least one sensor configured for measuring a swelling of the joint is a strain sensor which is adapted for at least partial wrapping around the joint such that a change of girth of the joint is detected as a change of strain.

4. Wearable device according to any one of claims 1 to 3, **characterized in that** the at least one sensor configured for measuring an orientation and/or a motion of the first body part relative to the second body part comprises motion detectors which are located in at least two of the three bandage sections.

5. Wearable device according to claim 4, **characterized in that** the motion detectors are configured as accelerometers and/or gyroscopes.

6. Wearable device according to claim 1, **characterized in that** the wearable device comprises at least one sensor configured for measuring the temperature of the joint and/or at least one sensor configured for measuring heart rate and/or at least one sensor configured for measuring blood pressure and/or at least one sensor configured for measuring oxygen saturation and/or at least one sensor configured for measuring activity.

7. Wearable device according to any one of claims 1 to 6, **characterized in that** the wearable device further comprises an input device configured to allow a patient to input a value representing a perceived pain, wherein the input device is connected to the communication device and the communication device is configured for wireless transmission of the value.

8. Wearable device according to claim 7, **characterized in that** the input device is configured as a slider.

9. Wearable device according to any one of claims 1 to 8, **characterized in that** the bandage comprises a wound dressing which is detachably connected to the third section of the bandage and configured to protect and/or treat a wound at the joint.

10. Wearable device according to any one of claims 1 to 9, **characterized in that** the communication device is adapted to communicate via wlan, Bluetooth, zigbee, ANT+ and/or cellular networks and is configured to communicate with an application executed on a smart device or a remote device accessible over the internet.

11. Wearable device according to any one of claims 1 to 10, **characterized in that** the bandage is configured as a bandage for the knee, the ankle, the hip, the wrist, the elbow or the shoulder of the human body in case of a human patient and is configured as a bandage for the knee, the fetlock joint or the hock in case of a horse as animal patient.

12. System comprising a wearable device according to any one of claims 1 to 11, wherein the bandage of the wearable device is configured as a bandage for a joint of a lower extremity of the human body, the system further comprising a sole insert comprising a sensor configured for measuring pressure distribution and the system being configured for determining a gait pattern using the measured pressure distribution and the system further comprising computing means being configured to determine a weight loading of the joint of the lower extremity, wherein the gait pattern is used as input value.

13. Method for monitoring the condition of a joint of a human or animal body, wherein a wearable device according to any one of claims 1 to 11 or a system according to claim 12 is worn by a patient or an animal prior to an intervention for a first time period, wherein reference data is acquired, the reference data at least including a reference range of motion of the joint and a reference swelling of the joint, and a wearable device according to any one of claims 1 to 11 or a system according to claim 12 is worn by the patient or an animal after an invention for a second time period and/or a third time period, wherein during the second time period and/or third time period sensor data which is characteristic for the condition of the joint is acquired and the condition of the joint is evaluated by comparing the acquired data with the previously acquired reference data, wherein the acquired data at least includes a range of motion of the joint derived from data acquired by means of motion sensors and a swelling of the joint.

14. Method according to claim 13, **characterized in that** at least during the second time period and/or the third time period further data is acquired and is considered for evaluating the condition of the joint, the further data being selected from heart rate, blood pressure, oxygen saturation, temperature of the joint, perceived pain, activity and combinations of at least two of these parameters.

15. Method according to claim 13 or 14, **characterized in that** acquired data and/or the evaluated condition of the joint is transmitted to a smart device of the patient and/or to a remote device.

16. Use of a wearable device according to any one of claims 1 to 11 for monitoring a joint in surgical and/or non-surgical treatment of a joint.
